# EUROPÄISCHE PATENTSCHRIFT

(11) **EP 3 288 532 B1**
(45) Veröffentlichungstag und Bekanntmachung des Hinweises auf die Patenterteilung: **23.06.2021**
(21) Anmeldenummer: 16717360.8
(22) Anmeldetag: 18.04.2016
(51) Int. Cl.: A61K 8/81, C08F 226/10, C08F 226/00

(54) **POLYMERE ZUR STABILISIERUNG PEROXIDISCHER VERBINDUNGEN**
POLYMERS FOR THE STABILISATION OF PEROXIDE COMPOUNDS
POLYMÈRES POUR LA STABILISATION DES COMPOSES PEROXYDIQUES

(30) Priorität: 28.04.2015 EP 15165457; 28.04.2015 EP 15165458
(43) Veröffentlichungstag der Anmeldung: 07.03.2018
(73) Patentinhaber: BASF SE, 67056 Ludwigshafen (DE)
(72) Erfinder: SCHADE, Christian, 67063 Ludwigshafen (DE); NATALELLO, Adrian, 68309 Mannheim (DE); DENUELL, Wolfgang, 68163 Mannheim (DE); MAMPE, Dirk, 40225 Düsseldorf (DE)
(74) Vertreter: BASF IP Association
(86) Internationale Anmeldenummer: PCT/EP2016/058499
(87) Internationale Veröffentlichungsnummer: WO 2016/173869

(56) Entgegenhaltungen:
- EP-A1- 1 647 267
- WO-A2-2012/019688
- WO-A2-2012/123241
- GB-A- 1 205 325
- US-A- 3 480 557
- US-A1- 2005 063 921
- US-A1- 2006 019 835
- US-A1- 2008 145 321
- US-A1- 2013 123 147

## Beschreibung

Die vorliegende Erfindung betrifft die Verwendung eines bestimmten, generisch definierten Copolymers zur Stabilisierung einer peroxidischen Verbindung gegen Zersetzung. Weiterhin betrifft die vorliegende Erfindung die Verwendung einer Zusammensetzung enthaltend eine peroxidische Verbindung und dieses bestimmte Copolymer zum Bleichen von Zähnen. Weiterhin betrifft die vorliegende Erfindung eine besondere Ausführungsform des Copolymers, nämlich ein Copolymer enthaltend Wiederholungseinheiten abgeleitet von N-Vinylpyrrolidon und mindestens einem Comonomer, das ausgewählt wird aus der Gruppe bestehend aus Vinylphosphonsäure, 2-Acrylamido-2-methylpropansulfonsäure und Kombinationen aus diesen Monomeren.

Es ist bekannt, Wasserstoffperoxid (im Folgenden auch mit H₂O₂ oder H₂O₂ bezeichnet) als Bleichmittel zu verwenden, um den Zahnschmelz aufzuhellen und also die Zähne zu bleichen, im Englischen auch als "teeth-whitening" bezeichnet. H₂O₂ ist in wässrigen Formulierungen bei Raumtemperatur nicht stabil. Deshalb sind Zahncremes, die H₂O₂ enthalten, üblicherweise wasserfrei, um eine vorzeitige Zersetzung des H₂O₂ während der Lagerung zu vermeiden oder mindestens zu verlangsamen. Bekannt sind zum Beispiel Zahncremes, die eine Zusammensetzung enthaltend Harnstoff und H₂O₂ enthalten, die auch als Harnstoff-H₂O₂ Komplex bezeichnet wird. Außerdem sind Zahncremes bekannt, die eine Zusammensetzung enthalten, welche Polyvinylpyrrolidon (PVP) und H₂O₂ enthält. Derartige Zusammensetzungen enthaltend ein Polymer und H₂O₂ werden oft als "Komplex aus Polymer und H2O2" bezeichnet. Im Allgemeinen ist die Stabilität von PVP-H2O2-Komplexen höher als die Stabilität der Harnstoff-H2O2 Komplexe. Gemeint ist jeweils die Stabilität gegen Zersetzung des H2O2.

Polymer-H₂O₂-Komplexe sind auch aus anderen Anwendungsgebieten als der Zahnpflege oder dem Bleichen von Zähnen bekannt.

WO 2011/130370 A1 beschreibt Copolymere mit einer Molmasse Mw von 500 bis 15000 D aus einem Vinylmonomer mit einer Amidgruppe und einem Vinylmonomer mit einer Säuregruppe oder dem entsprechenden Salz. Als mögliche Anwendungen für die Polymere werden Ölfeld-Anwendungen oder Korrosionsschutz offenbart. Die Verwendung dieser Copolymere für H₂O₂-Komplexe oder für die Zahnpflege wird nicht beschrieben.

DE 4344131 beschreibt die Herstellung von Polymer-H₂O₂-Komplexen durch Wirbelschichttrocknung. Als Polymere werden ausdrücklich erwähnt Copolymere aus N-Vinylcaprolactam und (Meth)acrylamidopropyl-3-sulfonsäure und Copolymere von N-Vinylpyrrolidon und Methacrylsäure im Gewichtsverhältnis 20:1 bis 1:20. Es wird beschrieben, dass diese Polymere als Desinfektionsmittel oder als Konservierungsmittel für die Zahnpflege eingesetzt werden können. Es wird nicht klar offenbart, was desinfiziert oder konserviert werden kann, ein Zahnpflegemittel oder Zähne.

WO 01/68045 beschreibt wasserfreie Klebestreifen zur Zahn-Aufhellung, deren Klebschicht aus einem Peroxid, einem Peroxid-Stabilisator und einem glasartigen hydrophilen Polymer besteht. Als Polymere werden Polyvinylpyrrolidon und Poly(vinylpyrrolidon-co-vinylacetat) genannt.
US 2012/0058059 beschreibt eine einphasige Zahncreme, die als Bleichmittel H₂O₂-PVP-Komplexe enthält. Die PVP-Komponente ist vernetzt.

US 2005/0036956 beschreibt eine Zusammensetzung zur Zahnbleiche, die neben einem H₂O₂-PVP-Komplex eine Verbindung mit erhöhter Zahnaffinität aufweist. Diese Verbindung kann ein Polymer sein. Als Beispiel werden Polycarboxylate oder Vinylpyrrolidon-VinylacetatCopolymere genannt. Weitere Copolymere werden nicht genannt.

US 2012/0301522 beschreibt eine wässrige Zusammensetzung zur Zahnbehandlung, die einen Komplex aus H₂O₂ und einem Vinyllactampolymer enthält. Als Baustein für die Polymere werden N-Vinylpyrrolidon und eine Vielzahl weiterer Lactammonomere genannt.

US 2008/0145321 beschreibt ein Mittel zur Zahnbehandlung, das einen Komplex aus einem Peroxid wie H₂O₂ und einem Vinyllactampolymer enthält. Als Baustein für die Polymere werden zahlreiche Lactammonomere wie z. B. N-Vinylpyrrolidon (NVP) genannt. Als weitere Polymere werden Copolymere aus NVP und Vinylacetat oder Dimethylaminoethylmethacrylat beschrieben.

WO 2012/123241 offenbart eine Zusammensetzung enthaltend Wasserstoffperoxid und das Copolymer Aristoflex AVS. Aristoflex AVS wird in WO 2012/123241 beschrieben als Natriumsalz eines Copolymers aus Vinylpyrrolidon und 2-Acrylamido-2-methylpropansulfonsäure.

Bekannte Zahncremes zum Bleichen der Zähne, die einen PVP-H2O2 Komplex enthalten, wirken dadurch, dass sich der PVP-H2O2-Komplex im Mund im Kontakt mit Speichel auflöst und H₂O₂ freisetzt. Dabei wird das H₂O₂ nicht gezielt am Ort seiner gewünschten Wirkung, der Zahnoberfläche, freigesetzt. Es wäre daher wünschenswert, das H₂O₂ an der Zahnoberfläche freizusetzen, um es so gezielt am gewünschten Wirkort bereit zu stellen. Der vorliegenden Erfindung liegt daher die Aufgabe zugrunde, eine peroxidische Verbindung, insbesondere H2O2, durch solche Polymere gegen Zersetzung zu stabilisieren, die eine besondere Affinität zu Zahnoberflächen haben.

Damit wäre es möglich, entsprechende Zusammensetzungen enthaltend derartige Polymere und eine peroxidische Verbindung, insbesondere H2O2, zum Bleichen von Zähnen zu verwenden. Dabei würde, wegen der besonderen Affinität der Polymere zu Zahnoberflächen, die peroxidische Verbindung, insbesondere das H2O2, in verstärktem Umfang an der Zahnoberfläche freigesetzt, und so gezielt am gewünschten Wirkort bereitgestellt.

Diese Aufgabe wird gelöst durch die im Folgenden definierte Verwendung eines Copolymers, welche ein Gegenstand der vorliegenden Erfindung ist.

Ein Gegenstand der vorliegenden Erfindung ist die Verwendung eines Copolymers enthaltend
- Wiederholungseinheiten abgeleitet von mindestens einem ersten Monomer, das ausgewählt wird aus der Gruppe bestehend aus N-Vinylpyrrolidon (N-Vinyl-2-pyrrolidon), N-Vinyl-2-piperidon, N-Vinyl-3-methyl-pyrrolidinon, N-Vinyl-3-methylpiperidon, N-Vinyl-3-methylcaprolactam, N-Vinyl-4-methyl-pyrrolidinon, N-Vinyl-4-methyl-2-pyrrolidon, N-Vinyl-4-methyl-piperidon, N-Vinyl-4-methyl-caprolactam, N-Vinyl-5-methyl-pyrrolidinon, N-Vinyl-5-ethyl-2-pyrrolidon, N-Vinyl-4-methylpiperidon, N-Vinyl-3-ethyl-pyrrolidinon, N-Vinyl-4, 5-dimethyl-pyrrolidinon, N-Vinyl-5, 5-dimethyl-pyrrolidinon, N-Vinyl-3, 3,5-trimethyl-pyrrolidinon, N-Vinyl-5-methyl-5-ethyl-pyrrolidinon, N-Vinyl-3,4,5-trimethyl-3-ethyl-pyrrolidinon, N-Vinyl-6-methyl-2-piperidon, N-Vinyl-6-ethyl-2-piperidon, N-Vinyl-3, 5-dimethyl-2-piperidon, N-Vinyl-4, 4-dimethyl-2-piperidon, N-Vinyl-2-caprolactam, N-Vinyl-7-methylcaprolactam, N-Vinyl-7-ethyl-caprolactam, N-Vinyl-3, 5-dimethyl-caprolactam, N-Vinyl-4, 6-dimethylcaprolactam, N-Vinyl-3, 5,7-trimethyl-caprolactam, N-Vinyl-2-valerolactam, N-Vinyl-hexahydro-2-azepinon, N-Vinyloctahydro-2-azocinon, N-Vinyl octahydro-2-azoninon, N-Vinyl decahydro-2-azecinon und Kombinatinon aus diesen Monomeren,
- mindestens einem Comonomer, das ausgewählt wird aus der Gruppe bestehend aus einem Monomer, das mindestens eine Phosphorsäureester-Gruppe enthält, einem Monomer, das mindestens eine Phosphonsäure-Gruppe enthält, einem Monomer, das mindestens eine Sulfonsäure-Gruppe enthält, einer ethylenisch ungesättigten Dicarbonsäure, einem ethylenisch ungesättigten Dicarbonsäureanhydrid und Kombinatinon aus diesen Monomeren,
- optional mindestens einem weiteren radikalisch polymerisierbaren, ethylenisch ungesättigten Monomer, insbesondere mindestens einem weiteren radikalisch polymerisierbaren, ethylenisch ungesättigten Monomer, welches eine OH-Gruppe oder eine mit 1 bis 10 Ethylenoxideinheiten ethoxylierte OH-Gruppe hat, insbesondere 2-Hydroxyethylmethacrylat (HEMA)
- und optional mindestens einem vernetzenden Comonomer das mindestens zwei radikalisch polymerisierbare, ethylenisch ungesättigte Gruppen im Molekül hat,
- zur Stabilisierung einer peroxidischen Verbindung gegen Zersetzung,
- wobei diese peroxidische Verbindung ausgewählt wird aus der Gruppe bestehend aus einer organischen Peroxidverbindung und Wasserstoffperoxid, insbesondere ausgewählt wird aus der Gruppe bestehend aus einem organischen Hydroperoxid und Wasserstoffperoxid, insbesondere ausgewählt wird aus der Gruppe bestehend aus tert.-Butylhydroperoxid, Cumolhydroperoxid und Wasserstoffperoxid, und insbesondere Wasserstoffperoxid ist.

In einer Ausführungsform der genannten Verwendung ist das mindestens eine erste Monomer N-Vinylpyrrolidon.

In einer Ausführungsform der genannten Verwendung ist das mindestens eine erste Monomer N-Vinyl-2-caprolactam.

In einer Ausführungsform der genannten Verwendung wird das mindestens eine Comonomer ausgewählt aus der Gruppe bestehend aus Maleinsäure, Maleinsäureanhydrid, Fumarsäure, Vinylphosphonsäure, 2-Acrylamido-2-methylpropansulfonsäure, Acrylamidopropansulfonsäure und Kombinationen aus diesen Monomeren.

In einer Ausführungsform der genannten Verwendung wird das mindestens eine Comonomer ausgewählt aus der Gruppe bestehend aus Vinylphosphonsäure, 2-Acrylamido-2-methylpropansulfonsäure und Kombinationen aus diesen Monomeren.

In einer Ausführungsform der genannten Verwendung wird das mindestens eine vernetzende Comonomer ausgewählt aus der Gruppe bestehend aus N,N'-Divinylethylenharnstoff, N,N'-Divinylpropylenharnstoff, N,N'-Divinylimidazolid-2-on, N-Vinyl-2-ethyliden-pyrrolidon, N-Vinyl-3-ethyliden-pyrrolidon, Methylenbisacrylamid, einem Allylether, einem Vinylether, einem (Meth)Acrylester einem (Meth)acrylamid eines Alkohols oder eines Amins die mehr als zwei funktionelle Gruppen aufweisen, wobei der Alkohol bevorzugt ausgewählt wird aus der Gruppe bestehend aus Ethylenglycol, Diethylenglycol, Butandiol, Hexandiol, Trimethylolpropan, Pentaerythritol Polyethylenglycol, Polypropylenglycol, und alkoxylierten Derivaten der genannten Alkohole, und Kombinatinon aus diesen Monomeren.

In einer Ausführungsform der genannten Verwendung das Copolymer, bezogen auf die gesamte Masse der Wiederholungseinheiten des Copolymers
- 70 - 99,5 Gew.-%, bevorzugt 80 - 99 Gew.-%, bevorzugt 85 - 98 Gew.-% Wiederholungseinheiten abgeleitet von dem mindestens einen ersten Monomer,
- 0,5 - 30 Gew.-%, bevorzugt 1 - 20 Gew.-%, bevorzugt 2-15 Gew.-% Wiederholungseinheiten abgeleitet von dem mindestens einen Comonomer,
- 0 bis 20 Gew.-%, bevorzugt, 0 bis 10 Gew.-%, bevorzugt 0 bis 5 Gew.-%, bevorzugt 0 bis 2 Gew.-%, insbesondere 0 Gew.-% Wiederholungseinheiten abgeleitet von dem mindestens einen weiteren radikalisch polymerisierbaren, ethylenisch ungesättigten Monomer,
- 0 bis 10 Gew.-%, bevorzugt 0 bis 2 Gew.-%, bevorzugt 0 - 1 Gew.-% Wiederholungseinheiten abgeleitet von dem mindestens einen vernetzenden Comonomer,
- wobei die Summe der Gewichtsanteile der Wiederholungseinheiten abgeleitet von dem mindestens einen ersten Monomer und von dem mindestens einen Comonomer und von dem mindestens einen weiteren radikalisch polymerisierbaren, ethylenisch ungesättigten Monomer und von dem mindestens einen vernetzenden Comonomer 100 Gew.-% ist.

In einer Ausführungsform der genannten Verwendung
- Ist das mindestens eine erste Monomer N-Vinylpyrrolidon,
- und das mindestens eine Comonomer wird ausgewählt wird aus der Gruppe bestehend aus Vinylphosphonsäure, 2-Acrylamido-2-methylpropansulfonsäure und Kombinationen aus diesen Monomeren,
- und das Copolymer enthält, bezogen auf die gesamte Masse der Wiederholungseinheiten des Copolymers, 70 - 99,5 Gew.-%, bevorzugt 80 - 99 Gew.-%, bevorzugt 85 - 98 Gew.-% Wiederholungseinheiten abgeleitet von N-Vinylpyrrolidon, und 0,5 - 30 Gew.-%, bevorzugt 1 - 20 Gew.-%, bevorzugt 2 - 15 Gew.-% Wiederholungseinheiten abgeleitet von dem mindestens einen Comonomer, wobei die Summe der Gewichtsanteile der Wiederholungseinheiten abgeleitet von dem mindestens einen ersten Monomer und von dem mindestens einen Comonomer 100 Gew.-% ist.

In einer Ausführungsform der genannten Verwendung
- Ist das mindestens eine erste Monomer N-Vinyl-2-caprolactam,
- und das mindestens eine Comonomer wird ausgewählt wird aus der Gruppe bestehend aus Vinylphosphonsäure, 2-Acrylamido-2-methylpropansulfonsäure und Kombinationen aus diesen Monomeren,
- und das Copolymer enthält, bezogen auf die gesamte Masse der Wiederholungseinheiten des Copolymers, 70 - 99,5 Gew.-%, bevorzugt 80 - 99 Gew.-%, bevorzugt 85 - 98 Gew.-% Wiederholungseinheiten abgeleitet von N-Vinyl-2-caprolactam, und 0,5 - 30 Gew.-%, bevorzugt 1 - 20 Gew.-%, bevorzugt 2 - 15 Gew.-% Wiederholungseinheiten abgeleitet von dem mindestens einen Comonomer, wobei die Summe der Gewichtsanteile der Wiederholungseinheiten abgeleitet von dem mindestens einen ersten Monomer und von dem mindestens einen Comonomer 100 Gew.-% ist.

Ein weiterer Gegenstand der vorliegenden Erfindung ist die Verwendung einer Zusammensetzung enthaltend eine peroxidische Verbindung, wobei diese peroxidische Verbindung ausgewählt wird aus der Gruppe bestehend aus einer organischen Peroxidverbindung und Wasserstoffperoxid, insbesondere ausgewählt wird aus der Gruppe bestehend aus einem organischen Hydroperoxid und Wasserstoffperoxid, insbesondere ausgewählt wird aus der Gruppe bestehend aus tert.-Butylhydroperoxid, Cumolhydroperoxid und Wasserstoffperoxid, und insbesondere Wasserstoffperoxid ist, und ein Copolymer wie definiert in einem der oben stehenden Absätze, die den Gegenstand der vorliegenden Erfindung und besondere Ausfürhungsformen beschreiben, zum Bleichen von Zähnen, wobei diese Zusammensetzung bevorzugt durch Sprühtrocknung erhältlich ist, und wobei diese Zusammensetzung, pro 100 g Copolymer, bevorzugt 1 - 32 g, insbesondere 3 - 30 g, insbesondere 8 - 28 g peroxidische Verbindung enthält.

In einer Ausführungsform der genannten Verwendung ist die Zusammensetzung enthalten in einer Formulierung ausgewählt aus der Gruppe bestehend aus einer Zahncreme einer Mundspülung einem Gel zum Bleichen der Zähne und einem Mundspray, wobei die Zusammensetzung bevorzugt in einer solchen Menge in der Formulierung enthalten ist, dass die Formulierung 0,01 bis 5 Gew.-%, insbesondere 0,1 bis 4 Gew.-%, insbesondere 0,3 bis 3 Gew.-%, peroxidische Verbindung enthält.

In einer Ausführungsform der genannten Verwendung enthält die Formulierung weitere bekannte Hilfsstoffe und Inhaltsstoffe für diese Formulierungen. Für den Fall, dass die Formulierung eine Zahncreme ist, ist diese bevorzugt im Wesentlichen wasserfrei. im Wesentlichen wasserfrei bedeutet weniger als 8 Gew.-%, insbesondere weniger als 5 Gew.-%, insbesondere weniger als 3 Gew.-% Wasser. Die Zahncreme kann insbesondere, als Trägermaterial, Polyethylenoxid oder Polyethylenoxid-Polypropylenoxid-Copolymere enthalten.

Die Formulierung kann solche Hilfs- und Inhaltsstoffe enthalten wie dies offenbart wird in US 2012/0058059**.**

Ein weiterer Gegenstand der vorliegenden Erfindung ist ein Copolymer enthaltend Wiederholungseinheiten abgeleitet von einem ersten Monomer, das N-Vinylpyrrolidon ist, mindestens einem Comonomer, das ausgewählt wird aus der Gruppe bestehend aus Vinylphosphonsäure, 2-Acrylamido-2-methylpropansulfonsäure und Kombinationen aus diesen Monomeren, optional mindestens einem weiteren radikalisch polymerisierbaren, ethylenisch ungesättigten Monomer, und optional mindestens einem vernetzenden Comonomer das mindestens zwei radikalisch polymerisierbare, ethylenisch ungesättigte Gruppen im Molekül hat. Dabei enthält das Copolymer, bezogen auf die gesamte Masse der Wiederholungseinheiten des Copolymers.

In einer Ausführungsform des Copolymers, wie beschreiben in dem vorangehenden Absatz, wird das mindestens eine vernetzende Comonomer ausgewählt aus der Gruppe bestehend aus N,N'-Divinylethylenharnstoff, N,N'-Divinylpropylenharnstoff, N,N'-Divinylimidazolid-2-on, N-Vinyl-2-ethyliden-pyrrolidon, N-Vinyl-3-ethyliden-pyrrolidon, Methylenbisacrylamid, einem Allylether, einem Vinylether, einem (Meth)Acrylester einem (Meth)acrylamid eines Alkohols oder eines Amins, die mehr als zwei funktionelle Gruppen aufweisen, und Kombinatinon aus diesen Monomeren.

In einer Ausführungsform des Copolymers enthält das Copolymer, bezogen auf die gesamte Masse der Wiederholungseinheiten des Copolymers,
- 70 - 99,5 Gew.-% Wiederholungseinheiten abgeleitet von N-Vinylpyrrolidon, und
- 0,5 - 30 Gew.-% Wiederholungseinheiten abgeleitet von dem mindestens einen Comonomer,
- wobei die Summe der Gewichtsanteile der Wiederholungseinheiten abgeleitet von dem mindestens einen ersten Monomer und von dem mindestens einen Comonomer 100 Gew.-% ist

Ein weiterer Gegenstand der vorliegenden Erfindung ist eine Zusammensetzung enthaltend eine peroxidische Verbindung, wobei diese peroxidische Verbindung ausgewählt wird aus der Gruppe bestehend aus einer organischen Peroxidverbindung und Wasserstoffperoxid, insbesondere ausgewählt wird aus der Gruppe bestehend aus einem organischen Hydroperoxid und Wasserstoffperoxid, insbesondere ausgewählt wird aus der Gruppe bestehend aus tert.-Butylhydroperoxid, Cumolhydroperoxid und Wasserstoffperoxid, und insbesondere Wasserstoffperoxid ist, und ein Copolymer wie definiert in den vorangegangenen Absätzen, wobei diese Zusammensetzung bevorzugt durch Sprühtrocknung erhältlich ist, und wobei diese Zusammensetzung, pro 100 g Copolymer, bevorzugt 1 - 32 g, insbesondere 3 - 30 g, insbesondere 8 - 28 g peroxidische Verbindung enthält.

Ein weiterer Gegenstand der vorliegenden Erfindung ist eine Formulierung ausgewählt aus der Gruppe bestehend aus einer Zahncreme einer Mundspülung einem Gel zum Bleichen der Zähne und einem Mundspray enthaltend diese Zusammensetzung.

Das erfindungsgemäße Copolymer und das erfindungsgemäß zu verwendende Copolymer löst sich in gewissem Maße in Wasser (Speichel). Es hat eine gute Zahnaffinität (siehe QCM-Messungen im Beispielteil der vorliegenden Schrift). Damit werden die peroxidischen Verbindungen, insbesondere H2O2, verstärkt am Zahn freigesetzt, welches zu einem verstärkten Aufhellen der Zähne führt.

Beispiele für Monomere, die mindestens eine Phosphorsäure-Gruppe enthalten, sind Alkanol-Phosphorsäureester der Formel RO-P(=O)(OH)₂, wobei R eine Alkylgruppe, bevorzugt mit 1 bis 30 C-Atomen, ist.

Beispiele für Monomere, die mindestens eine Phosphonsäure-Gruppe enthalten, sind Vinylphosphonsäure, Phosphorsäuremonovinylester, Allylphosphonsäure, Phosphorsäuremonoallylester, 3-Butenylphosphonsäure, Phosphorsäure(mono-3-butenyl)ester, Phosphorsäuremono-(4-vinyloxybutyl)ester, Acrylsäure(phosphonoxyethyl) ester, Methacrylsäure(phosphonoxyethyl)ester, Phosphorsäuremono-(-2-hydroxy-3-vinyloxy-propyl)ester, Phosphorsäuremono-(1-phosphonoxymethyl-2-vinyloxy-ethyl)-ester, Phosphorsäuremono-(3-allyloxy-2-hydroxy-propyl)ester, Phosphorsäuremono-2-(allylox-1-phosphonoxymethyl-ethyl)ester, 2-Hydroxy-4-vinyloxymethyl-1,3,2-dioxaphosphol, 2-Hydroxy-4-allyloxymethyl-1,3,2-dioxaphosphol und Ester von Hydroxyethyl- oder Hydroxypropyl(meth)acrylat mit (Poly)Phosphorsäure.

Beispiele für Monomere, die mindestens eine Sulfonsäure-Gruppe enthalten, sind Allylsulfonsäure, Methallylsulfonsäure, Styrolsulfonsäure, Toluolsulfonsäure, Vinylsulfonsäure, Allyloxybenzolsulfonsäure oder 2-Acrylamido-2-methylpropansulfonsäure, 2-((Meth)acryloyl)ethylsulfonsäure, 2-Acrylamidomethyldodecylsulfonsäure und 3-(Meth)acryloxypropansulfonsäure.

Beispiele für ethylenisch ungesättigten Dicarbonsäuren sind Itakonsäure, Maleinsäure, Mesaconsäure, Citraconsäure, Fumarsäure, oder Methylenmalonsäure.

Beispiele für ethylenisch ungesättigten Dicarbonsäureanhydride sind Itakonsäure, Maleinsäure, Mesaconsäure, Citraconsäure, Fumarsäure, oder Methylenmalonsäure.

Die Widerholungseinheiten des Comonomeren können, wenn sie Säuregruppen enthalten, in dem erfindungsgemäßen Copolymer als freie Säuregruppen vorliegen oder sie können deprotoniert zusammen mit einem oder mehreren verschiedenen Kationen vorliegen. Kationen können zum Beispiel sein Alkalimetallkationen oder Ammoniumionen. Weitere geeignete Kationen werden durch Protonierung von Basen gebildet, beispielsweise von Ammoniak, organischen Aminen wie Aminomethylpropanol, Triethanolamin, Ethanolamin, hydrophob modifizierten Aminen wie N-(N,N-Bis-(hydroxyethyl)-aminopropyl)-N-hydroxyethyl-octadecylamin oder 9-Octadecenylamin oder natürlich vorkommenden Aminen wie Lysin, Histidin oder Cadaverin.

Das erfindungsgemäße Copolymer ist bevorzugt wasserlöslich. Wasserlöslich bedeutet, dass bei 20° C die Löslichkeit des erfindungsgemäßen Copolymer in Wasser mindestens 45 Gew.-% ist.

Das erfindungsgemäße Copolymer hat typischerweise einen K-Werte in einem Bereich von 10 bis 120, bevorzugt von 16 bis 60, besonders bevorzugt von 20 bis 45. Die K-Werte werden nach H. Fikentscher, Cellulose-Chemie 13, Seite 48 - 64 und Seite 71 - 94 (1932) gemessen (1% in 5 Gew.-%iger wässriger Kochsalz-Lösung bei 22 °C und pH 7). Der K-Wert ist ein Maß für die mittlere molare Masse eines Polymers. Je höher der K-Wert ist, desto höher ist die mittlere molare Masse.

Bei zu kleinen K-Werten liegen viele Endgruppen in dem erfindungsgemäßen Copolymer vor, die oxidiert werden können, was nachteilig ist. Bei zu hohen K-Werten können sich Schwierigkeiten bei der Sprühtrocknung der erfindungsgemäße Copolymer ergeben.

### Herstellung der erfindunasaemäßen Copolymere

Die erfindungsgemäßen Copolymere können durch radikalische Copolymerisation erhalten werden. Als Radikalstarter können übliche Azo-, Peroxy- sowie Redoxstarter eingesetzt werden. Bevorzugt werden H₂O₂/Kupfer-Starter oder 2,2'-Azobis(2-amidonopropan)dihydrochlorid eingesetzt. Die Reaktionstemperatur bei der Copolymerisation liegt üblicherweise zwischen 30 und 100 °C.

Die Herstellung der Copolymere kann nach dem Verfahren der Lösungspolymerisation in Wasser, in Alkoholen, wie z. B. Ethanol oder iso-Propanol, oder in Wasser/Alkohol-Mischungen durchgeführt werden. Bevorzugt wird die Copolymerisation in Wasser durchgeführt. Der Feststoffgehalt liegt dabei typischerweise zwischen 5 und 75 Gew.-%, bevorzugt zwischen 25 - 45 Gew.-%.

Das, ggf. vernetzte, erfindungsgemäße Copolymer kann nach verschiedenen Verfahren hergestellt werden, beispielsweise nach dem Verfahren der Gelpolymerisation - bevorzugt in wässrigem Medium -, oder nach dem Verfahren der inversen Emulsions- oder Suspensionspolymerisation, oder nach dem Verfahren der Wasser-in-Wasser Polymerisation in einem wässrigen Zweiphasengemisch. Ein weiteres Verfahren zur Herstellung des erfindungsgemäßen Copolymers ist eine Popcornpolymerisation der Monomere in Wasser als Lösungsmittel. Popcorn-Polymere sind vernetzt und wasserunlöslich. Sie können als gemahlene Partikel formuliert werden.

### Herstellung der Zusammensetzung enthaltend eine peroxidische Verbindung, insbesondere H₂O₂ und ein Copolymer (auch "Copolymer-Peroxid-Komplexe" genannt) gemäß der vorliegenden Erfindung

Als Peroxid (auch "peroxidische Verbindung" genannt) können organische Peroxide oder H₂O₂ eingesetzt werden. Bevorzugt werden Hydroperoxide wie tert.-Butylhydroperoxid oder Cumolhydroperoxid sowie H₂O₂ eingesetzt. Ganz besonders bevorzugt wird H₂O₂ eingesetzt. Das Peroxid wird bevorzugt als wässrige Lösung in einer Konzentration von 10 - 70 Gew.-%, bevorzugt 30 - 50 Gew.-%, eingesetzt.

Das Peroxid wird bevorzugt in Mengen von 1 - 40 Gew.-%, besonders bevorzugt 2 - 30 Gew.-%, ganz besonders bevorzugt 5 - 25 Gew.-%, bezogen auf den Copolymer-H₂O₂ Komplex, eingesetzt.

Bevorzugt wird eine wässrige Lösung eines erfindungsgemäßen Copolymers mit dem Peroxid vermischt und die erhaltene Lösung einem geeigneten Trocknungsverfahren unterzogen, beispielsweise Walzen-, Wirbelschicht-, Gefrier- oder Sprühtrocknung. Die Mischung der Lösungen kann dabei vor oder während des Trocknungsverfahrens erfolgen.

Bevorzugt erfolgt die Herstellung der Komplexe durch Sprühtrocknung einer Copolymer-H₂O₂ Lösung, nach dem Verfahren wie es in den Ansprüchen von EP 0 714 919 A2 beschrieben ist.

Wasserunlösliche Polymer-Peroxid-Komplexe können nach dem Verfahren der DE 19455380 A1 in wässriger Suspension mit H₂O₂ versetzt, abfiltriert und anschließend nach verschiedenen Verfahren getrocknet werden, z. B. in einem Schaufeltrockner.

H₂O₂ Komplexe der Popcornpolymere werden bevorzugt über das Wirbelschichtverfahren herstellen. Dabei wird das Polymer mit einer H₂O₂ Lösung besprüht und im definierten Luftstrom getrocknet. Die Temperatur der Zuluft beträgt üblicherweise zwischen 25 und 80°C und die Abluft 25 bis 70 °C. Detailliertere Beschreibungen finden sich insbesondere auf Seite 3 ab Zeile 47 in DE 4344131 A1. Mit Hilfe dieses Verfahrens lässt sich rieselfähiges Pulver gewinnen, welches einen Wassergehalt kleiner 5 Gew.-% hat.

### Verwendung

Die erfindungsgemäßen Copolymer-Peroxid-Komplexe werden bevorzugt in Zahnbehandlungsmitteln eingesetzt, insbesondere in Zahncremes. Außerdem werden Die erfindungsgemäßen Copolymer-Peroxid-Komplexe in Zahnbehandlungsmitteln eingesetzt ausgewählt aus der Gruppe bestehend aus einer Mundspülung einem Gel zum Bleichen der Zähne und einem Mundspray. Ganz besonders bevorzugt sind diese Zahnbehandlungsmittel im Wesentlichen wasserfrei. im Wesentlichen wasserfrei bedeutet weniger als 8 Gew.-%, insbesondere weniger als 5 Gew.-%, insbesondere weniger als 3 Gew.-% Wasser. Hohe Wassermengen verringern die Stabilität des Copolymer-Peroxid-Komplexes.

### Beispiele

Im Folgenden werden die folgenden Abkürzungen und Bezeichnungen verwendet:

| | |
|---|---|
| VP: | N-Vinylpyrrolidon |
| VCap: | N-Vinyl-2-caprolactam |
| AMPS: | 2-Acrylam ido-2-methylpropansulfonsäure |
| VPA: | Vinylphosphonsäure |
| Wako V 50: | 2,2'-Azobis(2-methylpropionamidin)dihydrochlorid (ein PolymerisationsInitiator) |
| K-Wert: | K-Wert nach Fikentscher |
| % bedeutet Gew.-%, wenn nichts anderes angegeben ist. | |

### Beispiel 1a: Copolymer VP-AMPS (im Massenverhältnis 90 zu 10), Semi-Batchfahrweise

Eine 2,5 Liter fassende Vorlage wurde mit einem Rührer, einer Kühleinheit, einem Innenthermometer und einer Dosierungsvorrichtung bestückt. In dieser wurde 920 g vollentsalztes Wasser (VE-Wasser), 0,55 g Ammoniak-Lösung (25 %-ig) sowie 256 g N-Vinylpyrrolidon (VP) vorgelegt und auf 75 °C Innentemperatur unter Stickstoffatmosphäre aufgeheizt. Das Initiatorsystem (27 g H₂O₂ (30 %-ig) in 138 g VE-Wasser und 0,12 g 0,09 %-ige Kupfer-II-chlorid-Lösung (CuCl₂-Lsg.)) wurde über 8 h über separate Vorratsgefäße in das Reaktionsgemisch dosiert. Beginnend mit der Initiatorzugabe wurde über einen Zeitraum von 4 h eine Mischung aus 464 g VP, 80 g 2-Acrylamido-2-methylpropansulfonsäure (AMPS), 30,85 g Natriumhydroxid (NaOH) und 56 g VE-Wasser kontinuierlich zudosiert. Nach Zugabe aller Reaktionspartner wurde das Reaktionsgemisch noch für weitere 2 h auf 75 °C Innentemperatur gehalten und daraufhin auf Raumtemperatur abgekühlt. Der Feststoffgehalt des Reaktionsproduktes betrug 43%. Die Analyse der 0,01 g/cm³ Polymerlösung (diese Polymerlösung ergab sich durch Verdünnung des erhaltenen Reaktionsgemisches) in einer 5 Gew.-% wässrigen Kochsalzlösung ergab einen K-Wert von 33.

### Beispiel 1b: Copolymer VP-AMPS (im Massenverhältnis 90 zu 10), K-Wert 66

Eine 2,5 Liter fassende Vorlage wurde mit einem Rührer, einer Kühleinheit, einem Innenthermometer und einer Dosierungsvorrichtung bestückt. In dieser wurden 920 g VE-Wasser, 0,55 g Ammoniak-Lösung (25 %-ig) sowie 256 g VP vorgelegt und auf 85 °C Innentemperatur unter Stickstoffatmosphäre aufgeheizt. 9 g des Initiators Wako V 50 wurden mit 157 g vermengt und in 5,5 h zum Reaktionsgemisch gegeben. Zeitgleich wurden über einen Zeitraum von 4 h eine Mischung aus 464 g VP, 80 g AMPS, 30,85 g NaOH und 56 g VE-Wasser kontinuierlich zudosiert. Zum gleichen Zeitpunkt beginnend wurde über ein weiteres Zulaufgefäß weitere 72 g 25 %-ige Ammoniak-Lösung innerhalb von 6,5 h zum Reaktionsgemisch zugegeben. Nach vollständiger Zugabe wurde für weitere 2 h die Innentemperatur auf 85 °C gehalten.
Das so erzeugte Produkt ist viskos, hat einen Feststoffgehalt von 44% und einen K-Wert von 66 (laut Analyse einer 0,01 g/cm³ Polymerlösung (diese Polymerlösung ergab sich durch Verdünnung des erhaltenen Reaktionsgemisches) in einer 5 Gew.-% wässrigen Kochsalzlösung).

### Beispiel 1c: Copolymer VP-AMPS (im Massenverhältnis 90 zu 10), Batchfahrweise

Eine 2,5 Liter fassende Vorlage wurde mit einem Rührer, einer Kühleinheit, einem Innenthermometer und einer Dosierungsvorrichtung bestückt. In dieser wurden 808 g VE-Wasser, 1,18 g Ammoniak-Lösung (25 %-ig) sowie 379,27 g VP und 84,2 g AMPS vorgelegt und auf 85 °C Innentemperatur unter Stickstoffatmosphäre aufgeheizt. Noch vor Erreichung der Zieltemperatur wurden aus drei unterschiedlichen Vorlagen 8,77 g H₂O₂ (30 %-ig), eine Mischung aus 1,05 g VE-Wasser und 0,08 g CuCl₂ sowie 1,71 g Ammoniak-Lösung (25 %-ig) in einem Zeitraum von 2 h zum Reaktionsgemisch gegeben. Anschließend wurde für weitere 2 h die Innentemperatur auf 85 °C konstant gehalten.
Das so erzeugte Produkt hat einen Feststoffgehalt von 33% und einen K-Wert von 31 (laut Analyse einer 0,01 g/cm³ Polymerlösung (diese Polymerlösung ergab sich durch Verdünnung des erhaltenen Reaktionsgemisches) in einer 5 Gew.-% wässrigen Kochsalzlösung).

### Beispiel 2: Copolymer VP-AMPS (im Massenverhältnis 95 zu 5)

Analog zu Beispiel 1a wurden 276 g N-Vinylpyrrolidon in der Vorlage und 484 g VP in der Monomermischung sowie 40 g AMPS eingesetzt. Die Monomermischung wurde über einen Zeitraum von 4 h zum Reaktionsgemisch zudosiert. Es wurde ein Copolymer mit 95 Gew.-% VP und 5 Gew.-% AMPS erhalten. Das erhaltene Produkt hat einen Feststoffgehalt von 42% und die Analyse der auf 0,01 g/cm³ verdünnten Polymerlösung in einer 5 Gew.-% wässrigen Kochsalzlösung ergab einen K-Wert von 38.

### Beispiel 3: Copolymer VP-VPA (im Massenverhältnis 90 zu 10)

Eine 2,5 Liter fassende Vorlage wurde mit einem Rührer, einer Kühleinheit, einem Innenthermometer und einer Dosierungsvorrichtung bestückt. In dieser wurden 950 g VE-Wasser, 74 g NaOH und 50 g Vinylphosphonsäure (VPA) vorgelegt. Unter Stickstoffatmosphäre wurde das Reaktionsgemisch auf 85 °C erwärmt. 450 g VP sowie eine Mischung aus 25 g Wako V 50 und 237,5 g VE-Wasser wurden in 8 h zum Reaktionsgemisch dosiert. Anschließend wurde für weitere 2 h die Innentemperatur auf 85°C konstant gehalten. So erhielt man eine gelbliche, viskose Lösung mit einem Feststoffgehalt von 28% und einem K-Wert von 36 (0,01 g/cm³ Polymerlösung in einer 5 Gew.-% wässrigen Kochsalzlösung).

### Beispiel 4: Copolymer VP-VPA (im Massenverhältnis 95 zu 5)

Die Reaktion wurde analog zu Beispiel 3 durchgeführt, wobei der VPA-Gehalt in der Vorlage auf 25 g sowie der NaOH Gehalt auf 25 g reduziert wurde. Die zu dosierende VP-Menge wurde auf 430 g erhöht, die Innentemperatur bei 85 °C konstant gehalten. Abschließend wurde auf Raumtemperatur abgekühlt.
Das erhaltene Produkt hat einen Feststoffgehalt von 31% und die Analyse der 0,01 g/cm³ Polymerlösung in einer 5 Gew.-% wässrigen Kochsalzlösung ergab einen K-Wert von 50.

### Bespiel 5: Sprühtrocknung des Polymers aus Beispiel 1a

1641 g einer 40 Gew.-% wässrigen Lösung eines Copolymers, welches 90 Gew.-% VP und 10 Gew.-% AMPS enthielt (gemäß Beispiel 1a), wurden mit 412 g einer 50 Gew.-% H₂O₂ Lösung sowie 300 g Wasser gemischt. Das erhaltene Gemisch wurde sprühgetrocknet. Die Zulufttemperatur des Stickstoffs betrug während der Sprühtrocknung 162-164 °C und die Ablufttemperatur wurde zwischen 75 °C und 78 °C gehalten. Der Durchsatz betrug 746 g/h. So wurde ein rieselfähiges Polymer erhalten.

### Bespiel 6: Sprühtrocknung des Polymers aus Beispiel 3

1001 g einer 30 Gew.-% wässrigen Lösung eines Copolymers, welche 90 Gew.-% VP und 10 Gew.-% VPA enthielt (gemäß Beispiel 3), wurden mit 174 g einer 50 Gew.-% H₂O₂ Lösung sowie 202 g Wasser gemischt. Das erhaltene Gemisch wurde sprühgetrocknet. Die Zulufttemperatur des Stickstoffs betrug während der Sprühtrocknung 155-160°C und die Ablufttemperatur wurde zwischen 68 °C und 70 °C gehalten. Der Durchsatz betrug 910 g/h. So wurde ein rieselfähiges Polymer erhalten.

### Untersuchung der Zahnaffinität

Die Zahnaffinität der sprühgetrockneten Polymer Polymere wurde mit Hilfe einer Quarzkristall-Mikrowaage (QCM) charakterisiert. Als Sensor wurde ein Quarzkristall verwendet, der mit nanokristallinem Hydroxylapatit beschichtet war (QSX 327 HA, Hersteller BioLin Scientific AB, Stockholm). Vor jedem Versuch wurde zunächst bei 36°C Wasser mit einer Rate von 50 ml/min über den Kristall geleitet und dessen Resonanzfrequenz gemessen. Anschließend wurde die Lösung des zu prüfenden Polymeren in Wasser (Konzentration: 50 ppm) - eingestellt auf einen pH-Wert von 7,0 - unter den gleichen Bedingungen über den Sensor geleitet. Eine Adsorption des Polymeren äußert sich in einer Abnahme der Resonanzfrequenz über die Zeit. Durch die so genannte Sauerbrey-Gleichung kann die gemessene Frequenzänderung in tatsächlich adsorbierte Massen bzw. Schichtdicken umgerechnet werden.

| **Polymer** | **Beispiel** | **Abnahme der Frequenz** |
|---|---|---|
| "Polyvinylpyrrolidon K-30" | Nicht erfindungsgemäß | 0 Hz |
| VP/AMPS Copolymer Molverhältnis 90 zu 10 | 1 | 3 Hz |
| VP/VPA Copolymer Molverhältnis 90 zu 10 | 3 | 3 Hz |

"Polyvinylpyrrolidon K-30" ist Luviskol® K30 PDR, erhältlich von der BASF SE.

Die Beispiele zeigen die Affinität der erfindungsgemäßen Polymere zu Zahnoberflächen.

### Weitere Beispiele

### Beispiel 7: Copolymer VCap-AMPS (im Massenverhältnis 90/10)

Eine 2,5 Liter fassende Vorlage wurde mit einem Rührer, einer Kühleinheit, einem Innenthermometer und einer Dosierungsvorrichtung bestückt. In dieser wurden 729 g VE-Wasser, 0,32 g Ammoniak-Lösung (25 %-ig) sowie 360 g VCap und 80 g AMPS vorgelegt und auf 85 °C Innentemperatur unter Stickstoffatmosphäre aufgeheizt. Noch vor Erreichung der Zieltemperatur wurden aus drei unterschiedlichen Vorlagen 27,72 g H₂O₂ (15 %-ig), eine Mischung aus 12,4 g VE-Wasser und 0,08 g CuCl₂ Lösung sowie 14,4 g Ammoniak-Lösung (2,5 %-ig) in einem Zeitraum von 2 h zum Reaktionsgemisch gegeben. Anschließend wurde für weitere 2 h die Innentemperatur auf 85 °C konstant gehalten.
Das so erzeugte Produkt hatte einen Feststoffgehalt von 29% und einen K-Wert von 16 (laut Analyse einer 0,01 g/cm³ Polymerlösung (diese Polymerlösung ergab sich durch Verdünnung des erhaltenen Reaktionsgemisches) in einer 5 Gew.-% wässrigen Kochsalzlösung).

Die VCap-AMPS Copolymer-Lösung wurde anschließend 1 zu 1 mit einer 50 Gew.% H₂O₂ Lösung versetzt und in eine Petrischale gegeben. Diese Lösung wurde für 12 h im Vakuumschrank bei 45 °C getrocknet. Auf diese Weise konnte ein transparenter VCap-AMPS-Copolymer-H₂O₂ Komplex-Film erhalten werden.

### Beispiel 8: Vorbeugung vor Verfärbung und Aufhellung eines bereits angefärbten Apatit Pulvers

Um die aufhellenden Eigenschaften und vorbeugenden Fähigkeiten vor Verfärbung des Copolymer-H2O2-Komplexes zu zeigen wurden folgende Experimente durchgeführt:
1. Kontrollversuch: Anfärbung von Apatit mit Kaffee
2. Vorbeugende Eigenschaften gegenüber Verfärbungen
3. Aufhellende Eigenschaften des verfärbten Apatits

Verwendete Chemikalien
- Sprühgetrockneter PVP-AMPS-Copolymer-H2O2-Komplex gemäß Beispiel 5
- Kaffee: Contal Gold Instant Kaffee 100% Arabica von der Penny Markt GmbH
- Apatit: Hydroxyapatit von Sigma Aldrich CAS: 1306-06-5

Durchführung der Verfärbungsexperimente:
Zu 1: 10 g Apatit wurden mit 30 mL Kaffee (2 Gew.%) 15 min bei 37 °C gerührt und anschließend zentrifugiert.
   Der Feststoff wurde dann mit 30 mL VE-Wasser gewaschen und zentrifugiert.
   Dann wurde der folgende Waschschritt zwei Mal durchgeführt: Der Feststoff wurde mit 30 mL VE-Wasser bei 37 °C für 15 min gerührt und abzentrifugiert.
   Abschießend wurde der Feststoff für 12 h bei 45 °C im Vakuumschrank getrocknet und dann gemörsert.
Zu 2: 10 g PVP-AMPS Copolymer H₂O₂ Komplex wurden in 30 g Wasser gelöst und mit 10 g Apatit vermengt. Dieses Gemisch wurde für 15 min gerührt und anschließend zentrifugiert. Anschließend wurde der Feststoff mit 30 mL Kaffee (2 Gew.%) 15 min bei 37 °C gerührt und zentrifugiert.
   Analog zu 1 wurde aufgearbeitet:
   Der Feststoff wurde mit 30 mL VE-Wasser gewaschen und zentrifugiert.
   Dann wurde der folgende Waschschritt zwei Mal durchgeführt: Der Feststoff wurde mit 30 mL VE-Wasser bei 37 °C für 15 min gerührt und abzentrifugiert.
   Abschießend wurde der Feststoff für 12 h bei 45 °C im Vakuumschrank getrocknet und dann gemörsert.
Zu 3: Verfärbung des Apatits analog zu 1:
   10 g Apatit wurden mit 30 mL Kaffee (2 Gew.%) 15 min bei 37 °C gerührt und anschließend zentrifugiert.
   Der Feststoff wurde dann mit 30 mL VE-Wasser gewaschen und zentrifugiert.
   Dann wurde der folgende Waschschritt zwei Mal durchgeführt: Der Feststoff wurde mit 30 mL VE-Wasser bei 37 °C für 15 min gerührt und abzentrifugiert.
   Der angefärbte Apatit wurde mit 10 g Polymer (PVP-AMPS Copolymer H₂O₂ Komplex) in 30 g VE-Wasser für 15 min bei 37 °C gerührt und anschließend zentrifugiert.
   Abschießend wurde der Feststoff für 12 h bei 45 °C im Vakuumschrank getrocknet und dann gemörsert

Beobachtungen bei 1, 2 und 3:
Alle drei Proben sind bräunlich gefärbt. Probe 1 (Kontrollversuch, mit Kaffee angefärbt) ist dunkler als Probe 2 (vorbeugend mit Copolymer H₂O₂ Komplex behandelt, vor der Anfärbung mit Kaffee). Probe 2 wiederum ist dunkler ist als Probe 3 (nach Anfärbung mit Kaffee mit Copolymer H₂O₂ Komplex behandelt). Diese Beispiele zeigen die Wirksamkeit des Copolymer-H2O2-Komplexes sowohl für vorbeugende Maßnahmen gegen ungewünschte Verfärbungen als auch für die Aufhellung bei bestehenden Verfärbungen. Apatit ist dem Zahnschmelz sehr ähnlich. Also kann davon ausgegangen werden, dass entsprechende aufhellende Wirkungen auch bei Zähnen erreicht werden können.

## Patentansprüche

1. Die Verwendung eines Copolymers enthaltend Wiederholungseinheiten abgeleitet von
mindestens einem ersten Monomer, das ausgewählt wird aus der Gruppe bestehend aus N-Vinylpyrrolidon (N-Vinyl-2-pyrrolidon), N-Vinyl-2-piperidon, N-Vinyl-3-methylpyrrolidinon, N-Vinyl-3-methyl-piperidon, N-Vinyl-3-methylcaprolactam, N-Vinyl-4-methyl-pyrrolidinon, N-Vinyl-4-methyl-2-pyrrolidon, N-Vinyl-4-methyl-piperidon, N-Vinyl-4-methyl-caprolactam, N-Vinyl-5-methyl-pyrrolidinon, N-Vinyl-5-ethyl-2-pyrrolidon, N-Vinyl-4-methyl-piperidon, N-Vinyl-3-ethyl-pyrrolidinon, N-Vinyl-4, 5-dimethyl-pyrrolidinon, N-Vinyl-5, 5-dimethyl-pyrrolidinon, N-Vinyl-3, 3,5-trimethylpyrrolidinon, N-Vinyl-5-methyl-5-ethyl-pyrrolidinon, N-Vinyl-3,4,5-trimethyl-3-ethyl-pyrrolidinon, N-Vinyl-6-methyl-2-piperidon, N-Vinyl-6-ethyl-2-piperidon, N-Vinyl-3, 5-dimethyl-2-piperidon, N-Vinyl-4, 4-dimethyl-2-piperidon, N-Vinyl-2-caprolactam, N-Vinyl-7-methyl-caprolactam, N-Vinyl-7-ethyl-caprolactam, N-Vinyl-3, 5-dimethyl-caprolactam, N-Vinyl-4, 6-dimethylcaprolactam, N-Vinyl-3, 5,7-trimethyl-caprolactam, N-Vinyl-2-valerolactam, N-Vinyl-hexahydro-2-azepinon, N-Vinyloctahydro-2-azocinon, N-Vinyl octahydro-2-azoninon, N-Vinyl decahydro-2-azecinon, und Kombinatinon aus
diesen Monomeren,
mindestens einem Comonomer, das ausgewählt wird aus der Gruppe bestehend aus einem Monomer, das mindestens eine Phosphorsäureester-Gruppe enthält, einem Monomer, das mindestens eine Phosphonsäure-Gruppe enthält, einem Monomer, das mindestens eine Sulfonsäure-Gruppe enthält, einer ethylenisch ungesättigten Dicarbonsäure, einem ethylenisch ungesättigten Dicarbonsäureanhydrid und Kombinatinon aus diesen Monomeren,
optional mindestens einem weiteren radikalisch polymerisierbaren, ethylenisch ungesättigten Monomer,
und optional mindestens einem vernetzenden Comonomer das mindestens zwei radikalisch polymerisierbare, ethylenisch ungesättigte Gruppen im Molekül hat,
zur Stabilisierung einer peroxidischen Verbindung gegen Zersetzung,
wobei diese peroxidische Verbindung ausgewählt wird aus der Gruppe bestehend aus einer organischen Peroxidverbindung und Wasserstoffperoxid.

2. Die Verwendung nach Anspruch 1, wobei das mindestens eine erste Monomer ausgewählt wird aus der Gruppe bestehend aus N-Vinylpyrrolidon, N-Vinyl-2-caprolactam und einer Kombination beider Monomere.

3. Die Verwendung nach einem der Ansprüche 1 bis 2, wobei das mindestens eine Comonomer ausgewählt wird aus der Gruppe bestehend aus Maleinsäure, Maleinsäureanhydrid, Fumarsäure, Vinylphosphonsäure, 2-Acrylamido-2-methylpropansulfonsäure, Acrylamidopropansulfonsäure und Kombinationen aus diesen Monomeren.

4. Die Verwendung nach einem der Ansprüche 1 bis 2, wobei das mindestens eine Comonomer ausgewählt wird aus der Gruppe bestehend aus Vinylphosphonsäure, 2-Acrylamido-2-methylpropansulfonsäure und Kombinationen aus diesen Monomeren.

5. Die Verwendung nach einem der Ansprüche 1 bis 4, wobei das mindestens eine vernetzende Comonomer ausgewählt wird aus der Gruppe bestehend aus N,N'-Divinylethylenharnstoff, N,N'-Divinylpropylenharnstoff, N,N'-Divinylimidazolid-2-on, N-Vinyl-2-ethyliden-pyrrolidon, N-Vinyl-3-ethyliden-pyrrolidon, Methylenbisacrylamid, einem Allylether, einem Vinylether, einem (Meth)Acrylester einem (Meth)acrylamid eines Alkohols oder eines Amins, die mehr als zwei funktionelle Gruppen aufweisen, und Kombinatinon aus diesen Monomeren.

6. Die Verwendung nach einem der Ansprüche 1 bis 5, wobei das Copolymer enthält, bezogen auf die gesamte Masse der Wiederholungseinheiten des Copolymers,
70 - 99,5 Gew.-% Wiederholungseinheiten abgeleitet von dem mindestens einen ersten Monomer,
0,5 - 30 Gew.-% Wiederholungseinheiten abgeleitet von dem mindestens einen Comonomer,
0 bis 20 Gew.-% Wiederholungseinheiten abgeleitet von dem mindestens einen weiteren radikalisch polymerisierbaren, ethylenisch ungesättigten Monomer,
0 bis 10 Gew.-% Wiederholungseinheiten abgeleitet von dem mindestens einen vernetzenden Comonomer,
wobei die Summe der Gewichtsanteile der Wiederholungseinheiten abgeleitet von dem mindestens einen ersten Monomer und von dem mindestens einen Comonomer und von dem mindestens einen weiteren radikalisch polymerisierbaren, ethylenisch ungesättigten Monomer und von dem mindestens einen vernetzenden Comonomer 100 Gew.-% ist.

7. Die Verwendung nach Anspruch 1,
wobei das mindestens eine erste Monomer ausgewählt wird aus der Gruppe bestehend aus N-Vinylpyrrolidon, N-Vinyl-2-caprolactam und einer Kombination beider Monomere,
und wobei das mindestens eine Comonomer ausgewählt wird aus der Gruppe bestehend aus Vinylphosphonsäure, 2-Acrylamido-2-methylpropansulfonsäure und Kombinationen aus diesen Monomeren,
und wobei das Copolymer enthält, bezogen auf die gesamte Masse der Wiederholungseinheiten des Copolymers,
70 - 99,5 Gew.-% Wiederholungseinheiten abgeleitet von dem mindestens einen ersten Monomer, und
0,5 - 30 Gew.-% Wiederholungseinheiten abgeleitet von dem mindestens einen Comonomer,
wobei die Summe der Gewichtsanteile der Wiederholungseinheiten abgeleitet von dem mindestens einen ersten Monomer und von dem mindestens einen Comonomer 100 Gew.-% ist.

8. Ein Copolymer enthaltend Wiederholungseinheiten abgeleitet von einem ersten Monomer, das N-Vinylpyrrolidon ist,
mindestens einem Comonomer, das ausgewählt wird aus der Gruppe bestehend aus Vinylphosphonsäure, 2-Acrylamido-2-methylpropansulfonsäure und Kombinationen aus diesen Monomeren,
optional mindestens einem weiteren radikalisch polymerisierbaren, ethylenisch ungesättigten Monomer,
und optional mindestens einem vernetzenden Comonomer das mindestens zwei radikalisch polymerisierbare, ethylenisch ungesättigte Gruppen im Molekül hat,
wobei das Copolymer enthält, bezogen auf die gesamte Masse der Wiederholungseinheiten des Copolymers,
70 - 99,5 Gew.-% Wiederholungseinheiten abgeleitet von dem mindestens einen ersten Monomer,
0,5 - 30 Gew.-% Wiederholungseinheiten abgeleitet von dem mindestens einen Comonomer,
0 bis 20 Gew.-% Wiederholungseinheiten abgeleitet von dem mindestens einen weiteren radikalisch polymerisierbaren, ethylenisch ungesättigten Monomer,
0 bis 10 Gew.-% Wiederholungseinheiten abgeleitet von dem mindestens einen vernetzenden Comonomer,
wobei die Summe der Gewichtsanteile der Wiederholungseinheiten abgeleitet von dem mindestens einen ersten Monomer und von dem mindestens einen Comonomer und von dem mindestens einen weiteren radikalisch polymerisierbaren, ethylenisch ungesättigten Monomer und von dem mindestens einen vernetzenden Comonomer 100 Gew.-% ist.

9. Das Copolymer nach Anspruch 8, wobei das mindestens eine vernetzende Comonomer ein solches ist wie definiert in Anspruch 5.

10. Das Copolymer nach Anspruch 8, wobei das Copolymer die Wiederholungseinheiten ein einer solchen Menge enthält wie definiert in Anspruch 7.

11. Eine Zusammensetzung enthaltend die peroxidische Verbindung wie definiert in Anspruch 1 und das Copolymer wie definiert in einem der Ansprüche 8 bis 10.

12. Eine Formulierung ausgewählt aus der Gruppe bestehend aus einer Zahncreme einer Mundspülung einem Gel zum Bleichen der Zähne und einem Mundspray enthaltend die Zusammensetzung nach Anspruch 11.

13. Ein Verfahren zur Stabilisierung einer peroxidischen Verbindung gegen Zersetzung umfassend das in Kontakt bringen der peroxidischen Verbindung mit einem Copolymer, wobei die peroxidische Verbindung und das Copolymer beide solche sind wie angegeben in den Ansprüchen 1 bis 7.

## Claims

1. The use of a copolymer comprising repeat units derived from
at least one first monomer which is selected from the group consisting of N-vinylpyrrolidone (N-vinyl-2-pyrrolidone), N-vinyl-2-piperidone, N-vinyl-3-methylpyrrolidinone, N-vinyl-3-methylpiperidone, N-vinyl-3-methylcaprolactam, N-vinyl-4-methylpyrrolidinone, N-vinyl-4-methyl-2-pyrrolidone, N-vinyl-4-methylpiperidone, N-vinyl-4-methylcaprolactam, N-vinyl-5-methylpyrrolidinone, N-vinyl-5-ethyl-2-pyrrolidone, N-vinyl-4-methylpiperidone, N-vinyl-3-ethylpyrrolidinone, N-vinyl-4,5-dimethylpyrrolidinone, N-vinyl-5,5-dimethylpyrrolidinone, N-vinyl-3,3,5-trimethylpyrrolidinone, N-vinyl-5-methyl-5-ethylpyrrolidinone, N-vinyl-3,4,5-trimethyl-3-ethylpyrrolidinone, N-vinyl-6-methyl-2-piperidone, N-vinyl-6-ethyl-2-piperidone, N-vinyl-3,5-dimethyl-2-piperidone, N-vinyl-4,4-dimethyl-2-piperidone, N-vinyl-2-caprolactam, N-vinyl-7-methylcaprolactam, N-vinyl-7-ethylcaprolactam, N-vinyl-3,5-dimethylcaprolactam, N-vinyl-4,6-dimethylcaprolactam, N-vinyl-3,5,7-trimethylcaprolactam, N-vinyl-2-valerolactam, N-vinylhexahydro-2-azepinone, N-vinyloctahydro-2-azocinone, N-vinyloctahydro-2-azoninone, N-vinyldecahydro-2-azecinone and combinations of these monomers,
at least one comonomer which is selected from the group consisting of a monomer comprising at least one phosphoric ester group, a monomer comprising at least one phosphonic acid group, a monomer comprising at least one sulfonic acid group, an ethylenically unsaturated dicarboxylic acid, an ethylenically unsaturated dicarboxylic anhydride and combinations of these monomers,
optionally at least one further free-radically polymerizable, ethylenically unsaturated monomer,
and optionally at least one crosslinking comonomer having at least two free-radically polymerizable, ethylenically unsaturated groups in the molecule,
for stabilization of a peroxidic compound against breakdown,
wherein this peroxidic compound is selected from the group consisting of an organic peroxide compound and hydrogen peroxide.

2. The use according to claim 1, wherein the at least one first monomer is selected from the group consisting of N-vinylpyrrolidone, N-vinyl-2-caprolactam and a combination of the two monomers.

3. The use according to either of claims 1 and 2, wherein the at least one comonomer is selected from the group consisting of maleic acid, maleic anhydride, fumaric acid, vinylphosphonic acid, 2-acrylamido-2-methylpropanesulfonic acid, acrylamidopropanesulfonic acid and combinations of these monomers.

4. The use according to either of claims 1 and 2, wherein the at least one comonomer is selected from the group consisting of vinylphosphonic acid, 2-acrylamido-2-methylpropanesulfonic acid and combinations of these monomers.

5. The use according to any of claims 1 to 4, wherein the at least one crosslinking comonomer is selected from the group consisting of N,N'-divinylethyleneurea, N,N'-divinylpropyleneurea, N,N'-divinylimidazolid-2-one, N-vinyl-2-ethylidenepyrrolidone, N-vinyl-3-ethylidenepyrrolidone, methylenebisacrylamide, an allyl ether, a vinyl ether, a (meth)acrylic ester, a (meth)acrylamide of an alcohol or of an amine having more than two functional groups, and combinations of these monomers.

6. The use according to any of claims 1 to 5, wherein the copolymer comprises, based on the total mass of the repeat units in the copolymer, 70%-99.5% by weight of repeat units derived from the at least one first monomer,
0.5%-30% by weight of repeat units derived from the at least one comonomer,
0% to 20% by weight of repeat units derived from the at least one further free-radically polymerizable, ethylenically unsaturated monomer,
0%-10% by weight of repeat units derived from the at least one crosslinking comonomer,
where the sum total of the proportions by weight of the repeat units derived from the at least one first monomer and from the at least one comonomer and from the at least one further free-radically polymerizable, ethylenically unsaturated monomer and from the at least one crosslinking comonomer is 100% by weight.

7. The use according to claim 1,
wherein the at least one first monomer is selected from the group consisting of N-vinylpyrrolidone, N-vinyl-2-caprolactam and a combination of the two monomers,
and wherein the at least one comonomer is selected from the group consisting of vinylphosphonic acid, 2-acrylamido-2-methylpropanesulfonic acid and combinations of these monomers,
and wherein the copolymer comprises, based on the total mass of the repeat units in the copolymer,
70%-99.5% by weight of repeat units derived from the at least one first monomer, and
0.5%-30% by weight of repeat units derived from the at least one comonomer,
where the sum total of the proportions by weight of the repeat units derived from the at least one first monomer and from the at least one comonomer is 100% by weight.

8. A copolymer comprising repeat units derived from a first monomer which is N-vinylpyrrolidone,
at least one comonomer which is selected from the group consisting of vinylphosphonic acid, 2-acrylamido-2-methylpropanesulfonic acid and combinations of these monomers,
optionally at least one further free-radically polymerizable, ethylenically unsaturated monomer,
and optionally at least one crosslinking comonomer having at least two free-radically polymerizable, ethylenically unsaturated groups in the molecule, wherein the copolymer comprises, based on the total mass of the repeat units in the copolymer,
70%-99.5% by weight of repeat units derived from the at least one first monomer,
0.5%-30% by weight of repeat units derived from the at least one comonomer,
0% to 20% by weight of repeat units derived from the at least one further free-radically polymerizable, ethylenically unsaturated monomer,
0%-10% by weight of repeat units derived from the at least one crosslinking comonomer,
where the sum total of the proportions by weight of the repeat units derived from the at least one first monomer and from the at least one comonomer and from the at least one further free-radically polymerizable, ethylenically unsaturated monomer and from the at least one crosslinking comonomer is 100% by weight.

9. The copolymer according to claim 8, wherein the at least one crosslinking comonomer is one as defined in claim 5.

10. The copolymer according to claim 8, wherein the copolymer comprises the repeat units in such an amount as defined in claim 7.

11. A composition comprising the peroxidic compound as defined in claim 1 and the copolymer as defined in any of claims 8 to 10.

12. A formulation selected from the group consisting of a toothpaste, a mouthwash, a gel for tooth bleaching and an oral spray, comprising the composition according to claim 11.

13. A method of stabilizing a peroxidic compound against breakdown, comprising the contacting of the peroxidic compound with a copolymer, wherein the peroxidic compound and the copolymer are both those as specified in claims 1 to 7.

## Revendications

1. Utilisation d'un copolymère contenant des motifs répétitifs issus
d'au moins un premier monomère qui est choisi dans le groupe constitué par N-vinylpyrrolidone (N-vinyl-2-pyrrolidone), N-vinyl-2-pipéridone, N-vinyl-3-méthyl-pyrrolidinone, N-vinyl-3-méthyl-pipéridone, N-vinyl-3-méthylcaprolactame, N-vinyl-4-méthyl-pyrrolidinone, N-vinyl-4-méthyl-2-pyrrolidone, N-vinyl-4-méthyl-pipéridone, N-vinyl-4-méthyl-caprolactame, N-vinyl-5-méthyl-pyrrolidinone, N-vinyl-5-éthyl-2-pyrrolidone, N-vinyl-4-méthyl-pipéridone, N-vinyl-3-éthyl-pyrrolidinone, N-vinyl-4,5-diméthyl-pyrrolidinone, N-vinyl-5,5-diméthyl-pyrrolidinone, N-vinyl-3,3,5-triméthyl-pyrrolidinone, N-vinyl-5-méthyl-5-éthyl-pyrrolidinone, N-vinyl-3,4,5-triméthyl-3-éthyl-pyrrolidinone, N-vinyl-6-méthyl-2-pipéridone, N-vinyl-6-éthyl-2-pipéridone, N-vinyl-3,5-diméthyl-2-pipéridone, N-vinyl-4,4-diméthyl-2-pipéridone, N-vinyl-2-caprolactame, N-vinyl-7-méthyl-caprolactame, N-vinyl-7-éthyl-caprolactame, N-vinyl-3,5-diméthyl-caprolactame, N-vinyl-4,6-diméthylcaprolactame, N-vinyl-3,5,7-triméthyl-caprolactame, N-vinyl-2-valérolactame, N-vinyl-hexahydro-2-azépinone, N-vinyloctahydro-2-azocinone, N-vinyloctahydro-2-azoninone, N-vinyldécahydro-2-azécinone et des combinaisons de ces monomères,
d'au moins un comonomère qui est choisi dans le groupe constitué par un monomère qui contient au moins un groupe ester d'acide phosphorique, un monomère qui contient au moins un groupe acide phosphonique, un monomère qui contient au moins un groupe acide sulfonique, un acide dicarboxylique éthyléniquement insaturé, un anhydride d'acide dicarboxylique éthyléniquement insaturé et une combinaison de ces monomères,
éventuellement d'au moins un monomère supplémentaire éthyléniquement insaturé, polymérisable par voie radicalaire,
et éventuellement d'au moins un comonomère réticulant qui possède au moins deux groupes éthyléniquement insaturés, polymérisables par voie radicalaire, dans la molécule, pour la stabilisation d'un composé peroxydique contre la décomposition,
ce composé peroxydique étant choisi dans le groupe constitué par un composé de type peroxyde organique et le peroxyde d'hydrogène.

2. Utilisation selon la revendication 1, l'au moins un premier monomère étant choisi le groupe constitué par N-vinylpyrrolidone, N-vinyl-2-caprolactame et une combinaison de ces deux monomères.

3. Utilisation selon l'une quelconque des revendications 1 et 2, l'au moins un comonomère étant choisi dans le groupe constitué par l'acide maléique, l'anhydride d'acide maléique, l'acide fumarique, l'acide vinylphosphonique, l'acide 2-acrylamido-2-méthylpropanesulfonique, l'acide acrylamidopropanesulfonique et des combinaisons de ces monomères.

4. Utilisation selon l'une quelconque des revendications 1 et 2, l'au moins un comonomère étant choisi dans le groupe constitué par l'acide vinylphosphonique, l'acide 2-acrylamido-2-méthylpropanesulfonique et des combinaisons de ces monomères.

5. Utilisation selon l'une quelconque des revendications 1 à 4, l'au moins un comonomère réticulant étant choisi dans le groupe constitué par N,N'-divinyléthylèneurée, N,N'-divinylpropylèneurée, N,N'-divinylimidazolid-2-one, N-vinyl-2-éthylidène-pyrrolidone, N-vinyl-3-éthylidène-pyrrolidone, méthylènebisacrylamide, un éther d'allyle, un éther de vinyle, un ester de (méth)acryle, un (méth)acrylamide d'un alcool ou d'une amine, qui présentent plus de deux groupes fonctionnels, et une combinaison de ces monomères.

6. Utilisation selon l'une quelconque des revendications 1 à 5, le copolymère contenant, par rapport à la masse totale des motifs répétitifs du copolymère,
70 à 99,5 % en poids de motifs répétitifs issus de l'au moins un premier monomère,
0,5 à 30 % en poids de motifs répétitifs issus de l'au moins un comonomère,
0 à 20 % en poids de motifs répétitifs issus de l'au moins un monomère supplémentaire éthyléniquement insaturé, polymérisable par voie radicalaire,
0 à 10 % en poids de motifs répétitifs issus de l'au moins un comonomère réticulant,
la somme des proportions en poids des motifs répétitifs issus de l'au moins un premier monomère et de l'au moins un comonomère et de l'au moins un monomère supplémentaire éthyléniquement insaturé, polymérisable par voie radicalaire et de l'au moins un comonomère réticulant étant de 100 % en poids.

7. Utilisation selon la revendication 1,
l'au moins un premier monomère étant choisi dans le groupe constitué par N-vinylpyrrolidone, N-vinyl-2-caprolactame et une combinaison de ces deux monomères, et l'au moins un comonomère étant choisi dans le groupe constitué par l'acide vinylphosphonique, l'acide 2-acrylamido-2-méthylpropanesulfonique et des combinaisons de ces monomères,
et le copolymère contenant, par rapport à la masse totale des motifs répétitifs du copolymère,
70 à 99,5 % en poids de motifs répétitifs issus de l'au moins un premier monomère, et
0,5 à 30 % en poids de motifs répétitifs issus de l'au moins un comonomère,
la somme des proportions en poids des motifs répétitifs issus de l'au moins un premier monomère et de l'au moins un comonomère étant de 100 % en poids.

8. Copolymère contenant des motifs répétitifs issus d'un premier monomère, qui est la N-vinylpyrrolidone, d'au moins un comonomère, qui est choisi dans le groupe constitué par l'acide vinylphosphonique, l'acide 2-acrylamido-2-méthylpropanesulfonique et des combinaisons de ces monomères,
éventuellement d'au moins un monomère supplémentaire éthyléniquement insaturé, polymérisable par voie radicalaire,
et éventuellement d'au moins un comonomère réticulant qui possède au moins deux groupes éthyléniquement insaturés, polymérisables par voie radicalaire, dans la molécule, le copolymère contenant, par rapport à la masse totale des motifs répétitifs du copolymère,
70 à 99,5 % en poids de motifs répétitifs issus de l'au moins un premier monomère,
0,5 à 30 % en poids de motifs répétitifs issus de l'au moins un comonomère,
0 à 20 % en poids de motifs répétitifs issus de l'au moins un monomère supplémentaire éthyléniquement insaturé, polymérisable par voie radicalaire,
0 à 10 % en poids de motifs répétitifs issus de l'au moins un comonomère réticulant,
la somme des proportions en poids des motifs répétitifs issus de l'au moins un premier monomère et de l'au moins un comonomère et de l'au moins un monomère supplémentaire éthyléniquement insaturé, polymérisable par voie radicalaire et de l'au moins un comonomère réticulant étant de 100 % en poids.

9. Copolymère selon la revendication 8, l'au moins un comonomère réticulant en étant un tel que défini dans la revendication 5.

10. Copolymère selon la revendication 8, le copolymère contenant les motifs répétitifs en une quantité telle que définie dans la revendication 7.

11. Composition contenant le composé peroxydique tel que défini dans la revendication 1 et le copolymère tel que défini dans l'une quelconque des revendications 8 à 10.

12. Formulation choisie dans le groupe constitué par un dentifrice, un rince-bouche, un gel pour blanchir les dents et un spray buccal contenant la composition selon la revendication 11.

13. Procédé pour la stabilisation d'une composition peroxydique contre la décomposition comprenant la mise en contact du composé peroxydique avec un copolymère, le composé peroxydique et le copolymère étant tous deux ceux indiqués dans les revendications 1 à 7.
